# EUROPEAN PATENT APPLICATION

(11) **EP 2 335 578 A1**
(43) Date of publication of application: **22.06.2011**
(21) Application number: 09180060.7
(22) Date of filing: 21.12.2009
(51) Int. Cl.: A61B 5/11, A61G 7/057

(54) **Sensor system**

(71) Applicant: Koninklijke Philips Electronics N.V., 5621 BA Eindhoven (NL)
(72) Inventor: Bruekers, Alphons, A, 5600 AE, Eindhoven (NL); Breebaart, Jeroen, 5600 AE, Eindhoven (NL); Boughhorbel, Sabri, 5600 AE, Eindhoven (NL)
(74) Representative: Damen, Daniel Martijn

(57) **Abstract**

The invention relates to a sensor system comprising a sensor array, the sensor array comprising a substrate layer and a plurality of individual first sensor elements for measuring a desired parameter, which first sensor elements are arranged on said substrate layer and define a sensor plane, wherein the sensor array further comprises one or more second sensor elements for measuring a further desired parameter,
and wherein the sensor system is configured to process sensor data from the first sensor elements in dependency of sensor data from the one or more second sensor elements.

## Description

### FIELD OF THE INVENTION

The invention relates to the field of sensor systems, and more specifically to flexible sensor systems used for monitoring a person, i.e. a living subject, in particular a young child or patient, positioned on the sensor system. The invention further relates to a method for processing sensor data received from a sensor system.

### BACKGROUND OF THE INVENTION

Sensor systems are widely known and widely used in an almost unlimited number of applications and application areas. One particular type of sensor system comprises a so-called Flexible Large-Area Sensor Carrier or FLASC. Such a sensor system is for example known from Tekscan, which produces the clinical BPMS™ system (Body Pressure Measurement System). Typically such a system comprises flexible polyester sheets on which electrically conductive electrodes are positioned such that the electrodes face each other. The electrodes are arranged in a patterned manner. The known BPMS™ system is a pressure sensitive resistive system wherein between the conductive electrodes a coating is provided. When pressure is applied on the coating, its electrical resistance is changed and thus pressure data can be obtained. Different types of pressure sensitive sensor systems are also known, such as capacitive systems and systems using for example optical fibers and photo diodes.

An example of a pressure sensor system is shown in United States patent document US 2009/0070939 in which a system for the prevention of pressure ulcers with patients is disclosed. The system comprises a flat cushioning pad in which an array of pressure transducers is provided. The system further comprises a processor that is arranged to collect the signals that originate from the sensors in the array. The processor executes software that analyses the signals from the sensors and may provide the results to a (computer) display showing a graphical map of the pressure data. The system may be provided with a cover member that is positionable on top of the cushioning pad with the array of sensors. In that case, the patient lies on the cover member during use of the system. The cover member is a thin porous cloth attached over a layer of moisture-absorbing material.

The material of the cloth is disposable and can be removed from the cushioning pad with the array of sensors.

Similarly, these sensor systems can also be used to monitor babies or young children in their bed or playpen. As an example, information that can be obtained by analysing pressure data from a baby on a pressure sensitive sensor system may include:
information on posture, e.g. lying on belly or on back, sitting, standing, etc.;
information on position and orientation, e.g. lying in the middle of the bed, or on the side, rotated, upside down, etc.;
information on activity level and type, e.g. sleeping quietly or moving often, and if moving, the type of movement (rotating, crawling, walking, etc.);
information on anthropometric properties, e.g. body size and weight;
information on vital signs, such as the breathing rate and body movement.

A concern of current sensor systems is that due to the many different situations that may arise during use of the sensor system, interpretation of the sensor data obtained by the sensor system is not detailed enough and/or the sensor data is distorted which reduces the possibility to gain useful information from the sensor data.

As an example, a nice property of most pressure sensitive sensor systems is that they are symmetric, which means that basically the same sensor data is obtained when the sensor system is used upside down. However, this means that information may be obtained indicating whether a baby or young child is lying on its side, but detailed information whether the baby or young child is on its right or left side can not be obtained.

Another example is based on a sensor system that can also be used as a kind of interface and which is symmetric as in the previous example. In this example, when sitting on the sensor system, touching the sensor system with the left hand may trigger action y, while touching the sensor system with the right hand will trigger action x. The functionality of a particular region of the sensor system is defined in reference to the position of the person on the sensor system. If the sensor system can also be used upside down, in principle, the functionality of the particular regions switches.

Yet another example is that multiple subjects, e.g. babies, may be placed on a sensor system. Detailed information about which data relates to which baby may be desired, but can not always be obtained by the current sensor systems. The same applies when a baby and a non-live object are placed on the sensor system.

A further example is that when the sensor system is a flexible sensor system, the sensor system may not be oriented perfectly flat, but may be bend or folded. A change in orientation of the sensor system may also be induced by a child playing on the sensor system. Especially, when the size of the sensor system does not comply with the size of the bed or playpen, the sensor system may be folded to fit, possibly around a mattress. This will distort the sensor data obtained by the sensor system and possibly reduces the interpretation possibilities of the data.

### SUMMARY OF THE INVENTION

It would be desirable to provide an improved sensor system with increased interpretation possibilities. It would also be desirable to provide an improved sensor system that can advantageously be used in more situations.

To better address one or more of these concerns, in a first aspect of the invention a sensor system is provided that comprises a sensor array, the sensor array comprising a substrate layer and a plurality of individual first sensor elements for measuring a desired parameter, which first sensor elements are arranged on said substrate layer and define a sensor plane, characterized in that, the sensor array further comprises one or more second sensor elements for measuring a further desired parameter, wherein the sensor system is configured to process sensor data from the first sensor elements in dependency of sensor data from the one or more second sensor elements.

By providing one or more second sensor elements, the sensor data produced by these second sensor elements can advantageously be used to interpret the sensor data from the first sensor elements in ways which are more difficult or impossible when the second sensor elements are absent. As an example, distortions may be detected by the one or more second sensor elements, so that corresponding sensor data from the first sensor elements can be ignored. Another example is that the sensor data can be combined to allow a more detailed interpretation of the sensor data of the first and/or the second sensor elements.

With the measurement of a parameter is meant a physical quantity such as temperature, pressure, humidity, etc.

In an embodiment, the sensor system comprises a control unit with a first input to receive the sensor data from the first sensor elements and a second input to receive the sensor data from the second sensor elements, and an output to provide an output signal based on the sensor data of the first sensor elements in dependency of the sensor data of the one or more second sensor elements. In this manner, both the sensor data from the first sensor elements and the sensor data from the second sensor elements is available in a single control unit. In this embodiment, the sensor data of the second sensor elements has been used to influence or manipulate the sensor data from the first sensor elements, for instance, only undistorted sensor data is contained in the output signal.

In an embodiment, the control unit comprises a second output for providing a second output signal based on the sensor data of the second sensor elements. Hence, the control unit is able to provide control on the basis of both the sensor data from the first sensor elements and the sensor data from the second sensor elements. In this embodiment, the sensor data of the second sensor elements may have been used to influence or manipulate the sensor data from the first sensor elements. However, the sensor data of both the first and second sensor elements is still available for future manipulations or combinations.

In an embodiment, the sensor array is a flexible large-area sensor carrier, which provides a cost-effective substrate for carrying the sensor array, i.e. the first and/or second sensor elements.

In an embodiment, the first sensor elements and/or the one or more second sensor elements comprise pressure sensitive elements, i.e. pressure sensors. The pressure sensitive elements are preferably chosen from a group comprising: pressure sensitive resistive elements, pressure sensitive capacitive elements, piezo-electric elements, piezoresistive elements, and optical elements. This provides a robust and cost-effective embodiment.

In an embodiment, the first sensor elements and/or one or more second sensor elements comprise accelerometers. Accelerometers can be advantageously used to determine an orientation of at least a part of the sensor system. In case the substrate layer of the sensor array is rigid, allowing minimal deformations, a single accelerometer can for instance be used to determine which side of the sensor array faces upwards, and which side faces downwards, by determining the gravitational direction. When the substrate layer is more flexible and also folding is allowed, more accelerometers may be used to determine the shape of the sensor plane. In principle, the more flexible the sensor system, the more accelerometers have to be used to accurately determine the shape of the sensor plane.

The accelerometers may be configured to determine accelerations, such as gravity, in one direction only. In a variant, the accelerometers may be configurred to determine accelerations in more than one direction, preferably in three orthogonal directions allowing to determine the local orientation of the sensor plane in more detail.

Accelerometers use gravity as a reference to determine the orientation of the sensor plane. However, also other references, such as the (earth) magnetic field or a light source may be used. It is also envisaged that an air flow is used as a reference, especially for upside down detection. When the sensor system is used in bed or a playpen, typically one side is facing a basis supporting the sensor system, and the other side is exposed to air. By determining which side of the sensor system experiences an air flow, it can be determined which side faces up and which side faces the basis.

The orientation of the sensor system can advantageously be used to determine on which side a living subject is lying or how a sensor system is folded, e.g. around a mattress. The accelerometers can also be used to detect movement, folding or a change in orientation. Depending on such detection, the sensor system may stop a process or start a new process, for instance by providing an alarm.

When the accelerometers can be used to determine the flatness of the sensor system, it may be possible to inform an user to properly adjust the sensor system. Non-flatness may be the result of folding, or it can be put on top of an object that may cause an uncomfortable situation or even injuries. The accelerometers can also be used to assess the shape of the surface where the sensor system is put on. For instance, based on the accelerometers, the shape of a bed may be determined. It may also be possible to detect that the sensor system is used in a sit, e.g. a children car sit.

Besides accelerometers, other sensors of which the output is dependent on their relative orientation with respect to gravity can be used as well, such as small form-factor switches including a small weight, or switches containing fluids wherein the fluid opens or closes an electric circuit (as also used in thermostats).

In an embodiment, the first sensor elements and/or the one or more second sensor elements comprise temperature sensors. Usually, the sensor elements which are not designed as temperature sensors still show an undesired dependency on temperature. The temperature sensors can now be used to calibrate the other sensor elements, or compensate the sensor data from the other sensor elements for temperature induced variations.

Temperature sensors can also be used to determine which sensor data from other sensor elements relates to a living subject and which sensor data relates to a non-live object.

Further, the sensor data from the temperature sensors can be used to determine which sensor data from the other sensor elements relates to which body part of a living subject on the sensor system. Possibly, the sensor data can also be used to determine if a body part is covered by clothing or not.

The temperature sensors can be used to check whether body parts of a baby are covered with a blank by measuring for instance the temperature of the head of the baby, which is advantageous since it is well-known that temperature regulation in babies is mainly facilitated by the head of the baby and a too high temperature of the head is a known risk factor for sudden death infant syndrome (SIDS). In such a case, the position of the head of the baby may be determined based on pressure data. Subsequently the temperature of the head is estimated from temperature sensors in the vicinity of the estimated head position. Hence, in this example, the signals from the temperature sensors are adjusted or selected based on pressure sensors. Thus according to the invention, in this example, the first sensor elements comprise or are the temperature sensors, and the pressure sensors are part of the second sensor elements. The reverse case is also possible, depending on the application and use of the sensor data.

In an embodiment, the first sensor elements and/or one or more second sensor elements comprise light detectors. Light detectors can be used to determine the orientation as the side facing upwards will generally be subjected to more light than the side facing downwards. The light detectors can also be used as an input or output for an interactive system.

In an embodiment, the first sensor elements and/or one or more second sensor elements comprise humidity sensors, which can be used to detect if a body part is wet, such as near the head (for vomitting) or any other body part (sweat or urine).

It is to be understood that an embodiment in which the first and/or second sensor elements comprise multiple types of sensors also falls under the scope of this invention. The first and/or second sensor elements may thus comprise accelerometers, temperature sensors, pressure sensors, etc. at the same time.

In an embodiment, each first sensor element has an associated second sensor element. Preferably, this association is spatially related, which means that a first sensor element is located at or near its associated second sensor element, at least in top view. This makes it simpler to combine the sensor data from the first and second sensor elements as the sensor data from a first sensor element and the corresponding second sensor element relate to the same area of the sensor plane.

The processing system, i.e. control system, is preferably configured to process sensor data from a first sensor element in dependency of sensor data from the associated second sensor element.

In an embodiment, one of the first or second sensor elements may be arranged at a perimeter of the sensor array. This is in particular advantageous for detecting the orientation of the sensor plane. For instance, a second sensor element may be provided at each corner of the sensor array. Another advantage may be that the wiring to the sensor elements is easier when the sensor elements are located at or near the perimeter of the sensor array.

In an embodiment, a plurality of first or second sensor elements may be provided forming a contour that follows the outer contour of the sensor array. By interpreting sensor data along such a contour, information may be obtained about the whole sensor array, for instance by interpolation. This embodiment is especially suitable when the second sensor elements are accelerometers for orientation detection.

When also sensor data distributed more evenly over the sensor plane is required, e.g. in case of temperature sensors or when the sensor system is highly flexible, a plurality of second sensor elements may be provided forming multiple contours within the outer contour of the sensor plane. These multiple contours may be equidistant to each other and/or concentric. It is noted that the property of concentric is not limited to circles, but may also apply to other shapes such as ovals or rectangles.

In an embodiment, the one or more second sensor elements are also arranged on the substrate layer. However, a separate substrate layer for the second sensor elements may also be provided. Preferably, the sensor plane defined by the second sensor elements is parallel to the sensor plane defined by the first sensor elements, more preferably both sensor planes are coplanar.

In an embodiment, the sensor system comprises a cover member removably positionable over at least the substrate layer, so as to cover the sensor plane. Preferably, the sensor system further comprises a combination of at least one detector and at least one data carrier that carries identification data relating to the cover member. The detector is provided on one of the sensor array and the cover member, and the data carrier is provided on the other one of the sensor array and the cover member. The data carrier is detectable by the detector, and the detector is further arranged to read said identification data.

By providing identification data that relates to the cover member, information is available that can identify the cover member that is positioned on the sensor array. Such identification of the cover member can for example be done by means of a code that is contained in said identification data, whereby that code is compared with a comparison table present in a control unit. With the availability of the information concerning the cover member that is used, it is possible to take that information into account when assessing the sensor data.

In an embodiment, the cover member has a support surface for supporting an object or living subject. Preferably, the cover member has a dimension in a direction substantially perpendicular to the support surface and comprises a material having a ratio between a Young's modulus of said material and said dimension that is between 1.10⁴ and 1.10⁷ Pa/m.

By providing a cover member that is characterized by said ratio of said dimension, which dimension normally will be its thickness, and the Young's modulus which is a mechanical property of the material the cover member is made of, a mechanical low-pass filter is provided. Such a low-pass filter has the effect that a load or pressure that is exerted by an object or living subject that is located on the support surface of the cover member is spread out over a larger area than the actual area of the object or living subject that faces the support surface. Hence, the maximum pressure exerted will have a reduced dependency on the actual shape and the position of the object or living subject. Furthermore, smaller objects are detectable and spatial aliasing will reduce as spreading of the pressure exerted will have the result that more sensor elements will detect a slowly spatially changing pressure pattern. As an additional advantage, the dynamic pressure range of the sensor elements can be reduced, as the spreading of the load will flatten the pressure profile and the maximum pressure value is reduced. As yet another advantage, the influence of temperature on pressure sensor is reduced, because of the thermal isolation properties of the cover member.

In an embodiment, the sensor system comprises an actuator for providing a feedback signal based on the sensor data from the first and/or second sensor elements. This feedback signal can be used as a warning or alarm signal when an undesired situation is detected, for instance a warning signal to a parent when the sensor system monitors a baby or young child. But it can also be used for a baby or young child to interact with the system, wherein a light or sound signal is provided based on an input from a living subject to the sensor system which input is detected by the first and/or second sensor elements.

In combination with the possibility to detect if a sensor system is upside down or not, the invention can also be used to assign different functionality to both sides of the sensor system. For example, one side of the sensor system is intended for gaming, whereas the other side serves as an alarm sensor by detecting a person standing on it. Another example is that one side is for babies up to 9 months where an alarm is given when the baby leaves the sensor system, whereas the other side is for toddlers up to three years of age that may leave the sensor system without warning.

According to another aspect of the invention, a method is provided for processing sensor data from a sensor system, said sensor system comprising a sensor array with a plurality of individual first sensor elements for measuring a desired parameter and defining a first sensor plane, and one or more second sensor elements for measuring a further desired parameter, wherein the sensor data from the first sensor elements is processed in dependency of sensor data from the one or more second sensor elements.

This method allows for a more versatile use of the sensor system and increases the interpretation possibilities for the sensor system.

In an embodiment, the sensor data from the one or more second sensor elements is used to calibrate the sensor data from the first sensor elements. As an example, the sensor data from the second sensor elements may be used to compensate for a parameter, e.g. temperature, measured by the second sensor elements that undesirably influences the sensor data of the first sensor elements.

In an embodiment, the sensor data from the one or more second sensor elements determines which sensor data from the first sensor elements is ignored or not. As an example, when the sensor system is folded around a mattresss, the sensor data from the first sensor elements located on the side and below the mattresss can not be used properly and may be ignored during interpretation, thereby reducing the chance of a wrong interpretation based on the sensor data.

In an embodiment, the sensor data from the one or more second sensor elements determines the orientation of the sensor array, wherein the sensor data from the first sensor elements is interpreted based on said orientation of the sensor array.

In an embodiment, the one or more second sensor elements are temperature sensors, wherein the sensor data from the one or more second sensor elements is used to determine which sensor data from the first sensor elements relates to a living subject and which sensor data relates to a non-live object.

In an embodiment, the one or more second sensor elements are temperature sensors, wherein the sensor data from the first and second sensor elements are combined to determine which sensor data relates to which body part of a living subject on the sensor array.

In an embodiment, an alarm signal is given when an undesired situation is detected by the first and/or second sensor elements.

In an embodiment, a feedback signal is generated based on an input from a living subject on the sensor array, which input is detected by the first and/or second sensor elements.

In an embodiment, the sensor data of the first and/or second sensor elements are stored for later retrieval, analysis or inspection.

The invention also relates to a mattress comprising a sensor sytem according to the invention. The invention further relates to a cloth being equipped with a sensor system according to the invention. Also, the invention relates to the use of a sensor system, mattress, or cloth according to the invention for monitoring a person, i.e. a living subject, in particular a young child or patient.

These and other aspects of the invention will be more readily appreciated as the same becomes better understood by reference to the following detailed description and considered in connection with the accompanying drawings in which like reference symbols designate like parts.

### BRIEF DESCRIPTION OF THE DRAWINGS

Figure 1 depicts an exploded view of an exemplary embodiment of a sensor system according to the invention;
Figure 2 depicts a schematic view of a pressure sensitive sensor array according to another embodiment of the invention;
Figure 3 depicts the use of a plurality of second sensor elements of a sensor system according to yet another embodiment in a first situation;
Figure 4 depicts the use of the plurality of second sensor elements of the sensor system of Fig. 3 in a second situation;
Figure 5 depicts the use of the plurality of second sensor elements of the sensor system of Fig. 3 in a third situation;
Figure 6 depicts the use of the plurality of second sensor elements of the sensor system of Fig. 3 in a fourth situation;
Figures 7A-7D depict different ways of positioning a plurality of second sensor elements on a sensor system according to the invention.

### DETAILED DESCRIPTION OF EMBODIMENTS

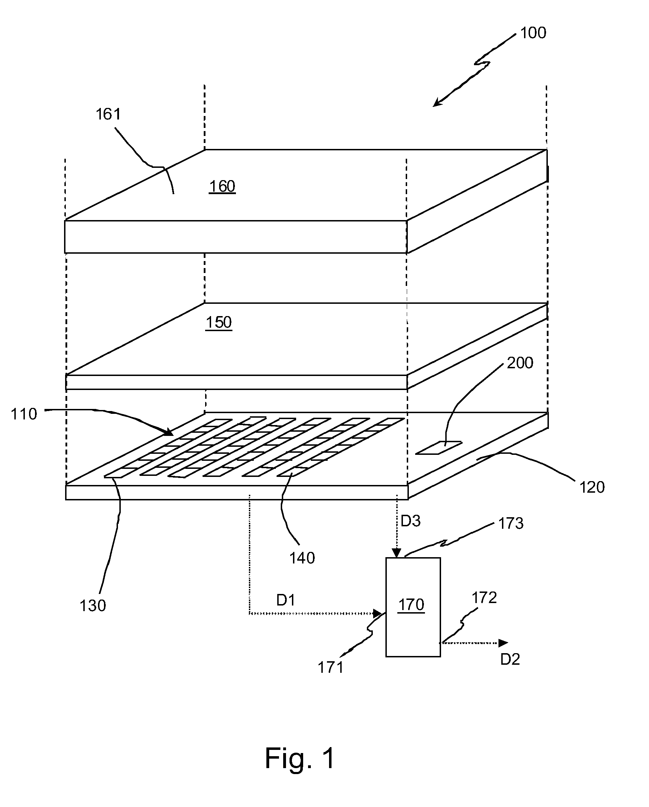

Figure 1 depicts a sensor system 100 according to an embodiment of the invention. Fig. 1 is an exploded schematic view of the sensor system 100. The sensor system 100 comprises a sensor array 110. The sensor array 110 comprises a first substrate layer 120 and a plurality of individual first sensor elements 130, in this case in the form of pressure sensitive elements. The first sensor elements 130 are arranged on the first substrate layer 120 to measure pressure exerted on the sensor array by an object or living subject (not shown) which may be positioned on a support surface 161. As can be seen, the first sensor elements 130 in the example shown define a substantially flat sensor plane 140.

The first sensor elements 130, in the example of Fig. 1, are covered by a second substrate layer 150 of similar dimensions as the first substrate layer 120. The provision of the second substrate layer 150 is not essential, but may be convenient for protecting the sensor elements 130.

In this example, the sensor array further comprises one second sensor element 200 for measuring a desired parameter. In this example, the second sensor element is an accelerometer to measure accelerations. With the accelerometer it is possible to determine the gravitational direction and possibly movement of the sensor array.

The sensor system is configured to process sensor data from the first sensor elements 130 in dependency of sensor data from the second sensor element 200.

The sensor system 100 comprises a control unit 170. The control unit 170 comprises a first input 171 for receiving a first input signal D 1 or sensor data generated by the first sensor elements 130 of the sensor array 110. The control unit 170 further comprises a second input 173 for receiving a second input signal D3 or sensor data generated by the second sensor element 200. The control unit 170 also comprises a first output 172 to provide a first output signal D2 comprising, at least part, of the sensor data of the first sensor elements 130. The first output signal D2 is based on the sensor data from the first sensor elements 130 in dependency of the sensor data of the second sensor element 200. The first output signal D2 may for example be supplied to a further control unit, a computer system or the like in which the output signal is further manipulated to be suitabel for display purposes on a display device for example.

The control unit 170 may also comprise a second output (not shown) to provide a second output signal based on the sensor data from the second sensor element.

The sensor system 100 of Fig. 1 further comprises a substantially flat cover member 160 that, in the example of Fig. 1, can be placed on top of the second substrate layer 150. However, in case the second substrate layer 150 is not present, the cover member 160 may be placed or arranged directly on the sensor array 110 and thus over at least the first substrate layer 120, so as to cover said sensor plane 140.

The accelerometer 200 of the sensor system 100 can be used to determine which side of the sensor array is facing upwards and which side is facing downwards. This information can be used to interpret the sensor data of the first sensor elements, and may be advantageous in case information about the exact posture is of a living subject on the sensor array is required, or when for instance information is required about which area of the sensor array corresponds to which functionality, e.g. induced by the left and/or right hand, when the sensor system is used in an interactive system.

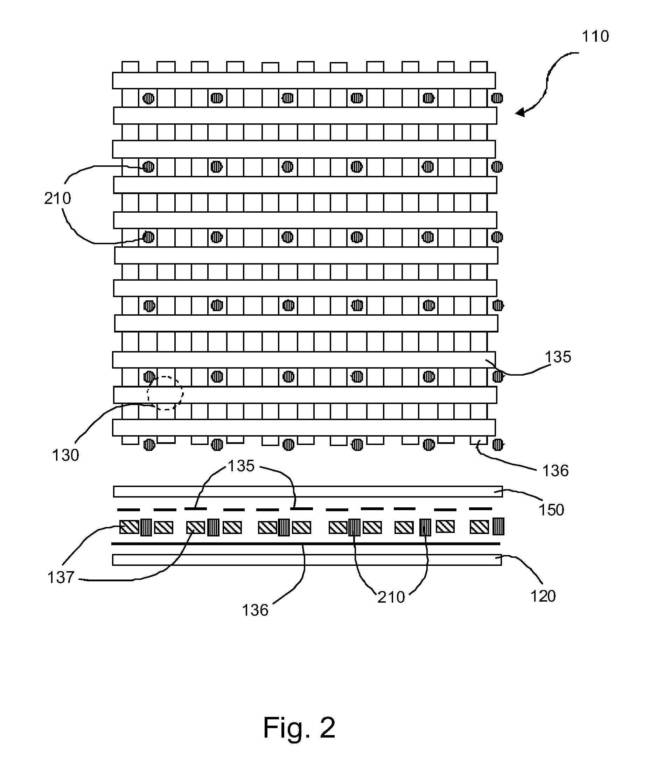

Fig. 2 (upper half) shows a schematic example of a sensor array 110 in plan view, comprising a plurality of first electrodes 135 that are attached to the second substrate layer 150 and a plurality of second electrodes 136 that are attached to the first substrate layer 120. As can be seen in Fig. 2, the first electrodes 135 and the second electrodes 136 are orthogonal with respect to each other. However, other patterns are also conceivable and the present invention is not limited to the example shown in Fig. 2.

The first and second electrodes 135, 136 are electrically conductive electrodes. Between the first electrodes and the second electrodes at those locations where said electrodes cross, pressure sensitive resistive material 137 is provided. This is shown in the lower half of Fig. 2, showing a schematic cross-sectional view.

The pressure sensitive resistive material 137 prevents direct contact between the first and second electrodes 135,136. The pressure sensitive resistive material 137 is for example a thin semi-conductive coating (ink), which material 137 has a varying electrical resistance when it is subjected to a varying pressure. The sensor array 110 hence comprises a plurality of pressure sensor elements 130 (indicated in Fig. 2 with a dotted circle) that are formed at each intersection of a first electrode 135 and a second electrode 136. The sensor array 110 is arranged to measure changes in current flow between the electrodes 135, 136 at each intersection when for example an object is placed on the second substrate layer 150. The measured changes in current flow (resulting from a change in the resistance of the pressure sensitive resistive material) represent the sensor data D1 (see Fig. 1).

A plurality of second sensor elements 210 is provided at a coarser grid in between the first and second substrate layer 120,150, wherein a second sensor element is located adjacent first and second electrodes 135, 136, i.e. adjacent first sensor elements 130. The second sensor elements in this example are shown highly schematic for simplicity reasons. It is to be understood that the second sensor elements although not shown are also connected to a control unit by wiring (not shown). The second sensor elements may be accelerometers to measure the orientation or shape of the sensor array 110, but may also be temperature sensors to measure temperature.

In the example of Fig. 2, the sensor system 100 utilizes pressure sensitive resistive material to form pressure sensitive sensor elements as first sensor elements. Other solutions are also known, such as pressure sensitive capacitive sensor elements, wherein the electrodes are separated by an air gap, and the distance between the electordes is a measure of the exerted pressure. Other solutions involve the use of piezo-electric elements, or optical elements. Such alternative solutions are known per se and to the person skilled in the art. An example of a so-called FLASC or Flexible Large-Area Sensor Carrier as schematically shown in Fig. 1 and Fig. 2 is the Tekscan BPMS™ system. The BPMS™ system may for example be used for monitoring patients in bed to avoid the occurrence of ulcers. Other applications are monitoring babies or young children in their bed or playpen.

As an example of an application of the sensor system of Fig. 2 with the second sensor elements being temperature sensors, is that combining the sensor data from the pressure sensors with the sensor data of the temperature sensors allows to determine which temperature measurements relate to e.g. the head of a child or e.g. the buttock of the child. In addition it is possible to deduce for instance whether a blanket covering the child is overthrown or covering the head, which may lead to respiratory problems and an increased head temperature.

From the temperature sensors it may also be possible to determine whether a body part is covered with clothes, e.g. socks. The information can also be combined with the sensor data from the pressure sensors to improve the accuracy of the detection of the head posture, because the temperature of the back of the head is less than the temperature of the front or the side of the head. It is also possible to raise an alarm if a very warm or hot object is detected in the presence of the baby.

Temperature sensors can be chosen from the group of temperature sensitive resistive material, thermoelectric sensing elements, thermometer, thermistor, etc., which are all known per se and to the skilled person in the art.

The temperature sensors can also be used to correct the sensor data from the pressure sensors for temperature variations.

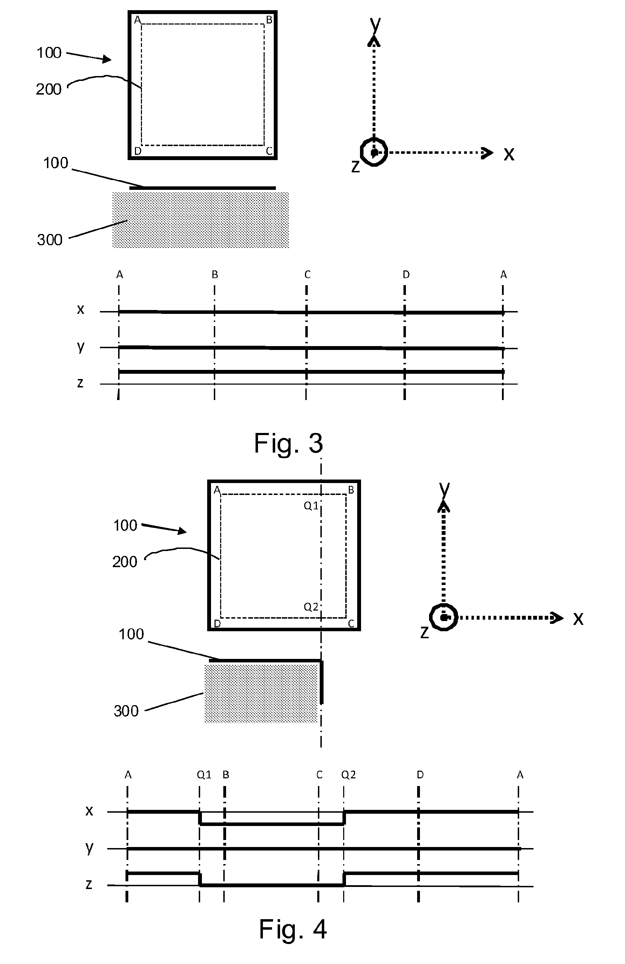

The Figs. 3-6 depict a sensor system 100 with a square shape in four different situations. At the top of each figure, the sensor system 100 is shown in top view. Next to the top view of the sensor system a coordinate system is defined in relationship to the top view. The coordinate system defines three orthogonal directions x, y, and z with respect to the sensor system itself, wherein z is directed out of plane. Below the top view of the sensor system, the situation is depicted in side view, wherein it can easily be seen how the sensor system is supported by a basis 300. At the bottom of each figure, sensor signals of a plurality of second sensor elements 200 provided on the sensor system are shown along a contour A-B-C-D-A as shown in the top view of the sensor system. The second sensor elements are in this case accelerometers which are able to measure accelerations in three orthogonal directions corresponding to the x, y, and z directions in the top view. The x, y, and z sensor signals of the second sensor elements thus show the measured accelerations in these directions.

In Fig. 3 the sensor system is flat and supported by the basis 300. As gravity is directed in the z-direction only and no other accelerations are applied, the x and y sensor signals are zero, and the z sensor signal shows a positive signal. The fact that the z sensor signal is positive in this orientation is a design choice and not essential.

It is possible that the sensor system is put upside down. This situation can be distinguished from the situation in Fig. 3 as the second sensor elements will output a negative z sensor signal, i.e. a z sensor signal opposite to the signal in Fig. 3. The ability to distinguish between the two situations, i.e. that the sensor system is upside down or upside up, can be used to:
process the sensor data from first sensor elements, so that the function of the sensor system is invariant to changes in the orientation of the sensor system, and/or,
assign different functionality to both sides of the sensor system, for instance when it is part of a user interface.

As examples of assigning different functionality:
one side may be intended for gaming, whereas the other side serves as an alarm sensor by detecting a person standing on it;
one side is for babies up to 9 months where an alarm is given when the baby leaves the sensor system, whereas the other side is for toddlers up to three years of age that may leave the sensor system without warning; or
one side is for use in a bed, whereas the other side is for use in a playpen.

In Fig. 4, the sensor system is folded along line Q1-Q2 over the edge of basis 300 as can be seen best in side view. For the portion of the sensor system folded over the side, gravity is directed in the x-direction of the second sensor elements 200 and not in the z-direction any more. So, for the contour portion Q1-B-C-Q2, the x sensor signal is non-zero, and the y and z sensor signals are zero.

Due to the different orientation, the sensor data from the first sensor elements in the portion of the sensor system that is oriented vertically may be useless. On the basis of the sensor signals of the second sensor elements, it can be determined which first sensor elements are useless, and subsequently, the sensor data from these sensors can be ignored in the interpretation of the sensor data.

It is also possible to inform a user to properly adjust the sensor system by sounding an alarm or using a light signal.

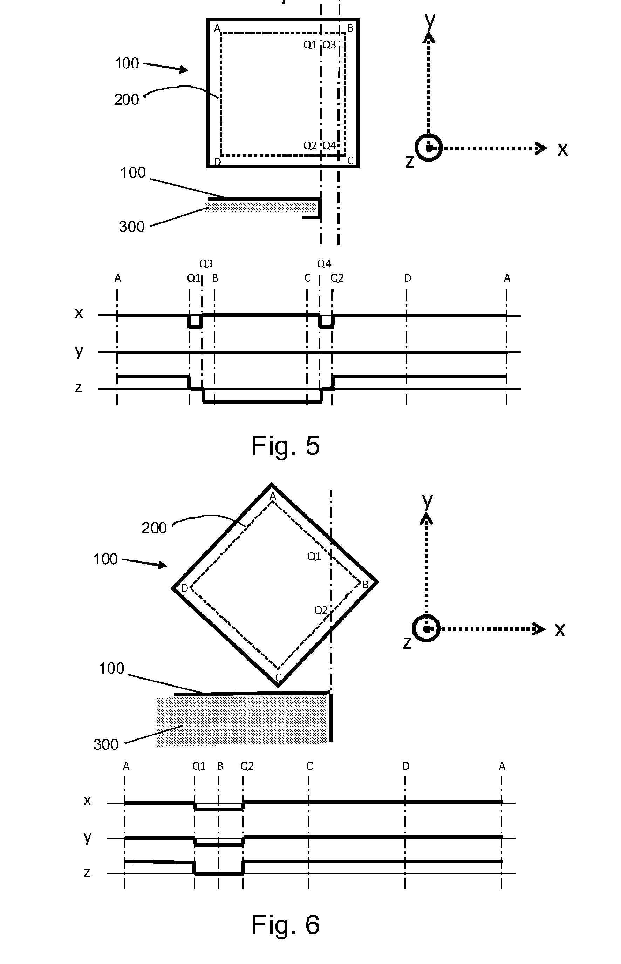

In Fig. 5, the sensor system is folded twice, once along line Q1-Q2, and once along line Q3-Q4, so that the sensor system is folded around the basis 300. This may for instance be the case when the sensor system is used in a bed but is too large for the mattress.

For the portion of the sensor system between the lines Q1-Q2 and Q3-Q4, gravity is directed in the x-direction. For the portion below the basis 300, gravity is directed in the z-direction, but this direction is opposite to the direction of gravity for the portion on top of the basis 300 as can also be seen in the sensor signals. Similarly to the example of Fig. 4, the sensor data of first sensor elements in the region at the right of the line Q1-Q2 (see the top view) can be ignored in interpretation. Alternatively, the sensor data from sensors in the region B-C-Q4-Q3 can be combined with data from sensors in the righthand side of region A-Q 1-Q2-D which overlap with the region B-C-Q4-Q3 in top view.

In Fig. 6, the sensor system is folded once along line Q1-Q2, but in this situation, a corner of the sensor system is folded over the edge of the basis 300. For the portion over the side, gravity is directed partially in the x-direction and partially in the y-direction as can be seen in the sensor signals. Thus, for the contour portion Q1-B-Q2, the x and y sensor signals are both non-zero.

As the second sensor elements of the embodiment according to Figs. 3-6 are able to measure accelerations in three orthogonal directions, the sensor data of the second sensor elements can be used to assess the shape of the surface where the sensor system is placed on.

It is further possible to detect movement of the sensor system, or that a change is made to the shape and/or orientation of the sensor system, which can occur due to a child playing on the sensor system, by analyzing the temporal behaviour of the signals originating from the sensor system, indicating movement.

It is also possible to detect that the sensor system is folded in two and then both portions are oriented horizontally and on top of each other. The sensor data of both the portions can still be used, but the sensor signals of one portion will be opposite to the sensor signals of the other portion due to the upside down orientation of one portion. Due to knowledge of the orientation of the sensor system, the sensor system is still able to properly process the sensor data of the first sensor elements. The advantage is that where the two portions overlap, the resolution of the sensor data has increased.

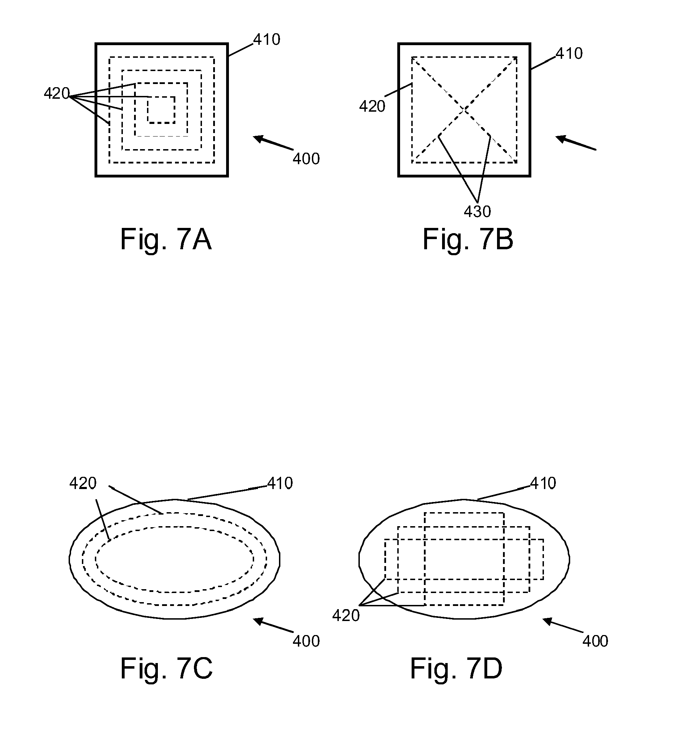

Fig. 7A-7D show different embodiments of a sensor system according to the invention and show different ways of positioning a plurality of second sensor elements on the sensor system.

In Fig. 7A a square sensor system 400 is shown with an outer contour 410. Within the outer contour, a plurality of second sensor elements are provided forming multiple contours 420 indicated by the dashed contours. In this example, the multiple contours are equidistant to each other and concentric.

In Fig. 7B, a square sensor system 400 is shown with an outer contour 410. Within the outer contour at the perimeter of the sensor system a contour 420 of a plurality of second sensor elements is shown. Additional second sensor elements are provided on the diagonals of the contour 420 and indicated by lines 430.

In Fig. 7C, an oval shaped sensor system 400 is shown with an outer contour 410. Within the outer contour of the sensor system two contours 420 are provided made of a plurality of second sensor elements, which contours 420 follow the outer contour 410. The two contours 420 are concentric and equidistant in this example.

In Fig. 7D is an oval shaped sensor system 400 shown with an outer contour 410. Within the outer contour of the sensor system, multiple contours 420 are provided which consist of a plurality of second sensor elements. In this example, the oval senor system is filled with rectangular contours to distribute the second sensor elements over the surface area of the sensor system. The rectangular contours are not equidistant, but are concentric with respect to each other.

As required, detailed embodiments of the present invention are disclosed herein; however, it is to be understood that the disclosed embodiments are merely exemplary of the invention, which can be embodied in various forms. Therefore, specific structural and functional details disclosed herein are not to be interpreted as limiting, but merely as a basis for the claims and as a representative basis for teaching one skilled in the art to variously employ the present invention in virtually any appropriately detailed structure. Further, the terms and phrases used herein are not intended to be limiting, but rather, to provide an understandable description of the invention.

The terms "a" or "an", as used herein, are defined as one or more than one. The term plurality, as used herein, is defined as two or more than two. The term another, as used herein, is defined as at least a second or more. The terms including and/or having, as used herein, are defined as comprising (i.e., open language, not excluding other elements or steps). Any reference signs in the claims should not be construed as limiting the scope of the claims or the invention.

The mere fact that certain measures are recited in mutually different dependent claims does not indicate that a combination of these measures cannot be used to advantage.

A single processor or other unit may fulfil the functions of several items recited in the claims.

The terms program, software application, and the like as used herein, are defined as a sequence of instructions designed for execution on a computer system. A program, computer program, or software application may include a subroutine, a function, a procedure, an object method, an object implementation, an executable application, an applet, a servlet, a source code, an object code, a shared library/dynamic load library and/or other sequence of instructions designed for execution on a computer system.

A computer program may be stored and/or distributed on a suitable medium, such as an optical storage medium or a solid-state medium supplied together with or as part of other hardware, but also be distributed in other forms, such as via the Internet or other wired or wireless telecommunication systems.

## Claims

1. A sensor system comprising a sensor array, the sensor array comprising a substrate layer and a plurality of individual first sensor elements for measuring a desired parameter, which first sensor elements are arranged on said substrate layer and define a sensor plane,
**characterized in that**,
the sensor array further comprises one or more second sensor elements for measuring a further desired parameter,
wherein the sensor system is configured to process sensor data from the first sensor elements in dependency of sensor data from the one or more second sensor elements.

2. A sensor system according to claim 1, comprising a control unit with a first input to receive the sensor data from the first sensor elements and a second input to receive the sensor data from the second sensor elements, and an output to provide an output signal based on the sensor data of the first sensor elements in dependency of the sensor data of the one or more second sensor elements.

3. A sensor system according to claim 1 or 2, wherein the sensor array is a flexible large-area sensor carrier.

4. A sensor system according to any of claims 1-3, wherein the first sensor elements or the one or more second sensor elements comprise pressure sensitive elements.

5. A sensor system according to any of claims 1-4, wherein the first sensor elements or the one or more second sensor elements comprise accelerometers.

6. A sensor system according to any of the claims 1-5, wherein the first sensor elements or the one or more second sensor elements comprise temperature sensors.

7. A sensor system according to any of the claims 1-6, wherein each first sensor element has an associated second sensor element which is preferably spatially related to the first sensor element.

8. A sensor system according to any of the claims 1-7, wherein a plurality of second sensor elements are provided forming a contour that follows the outer contour of the sensor array.

9. A sensor system according to any of the claims 1-8, wherein a plurality of second sensor elements are provided forming multiple contours within the outer contour of the sensor array.

10. A sensor system according to claim 9, wherein the multiple contours are concentric.

11. A method for processing sensor data from a sensor system, said sensor system comprising a sensor array with a plurality of individual first sensor elements for measuring a desired parameter and defining a first sensor plane, and one or more second sensor elements for measuring a further desired parameter, wherein the sensor data from the first sensor elements is processed in dependency of sensor data from the one or more second sensor elements.

12. A method according to claim 11, wherein the sensor data from the one or more second sensor elements is used to calibrate the sensor data from the first sensor elements.

13. A method according to claim 11 or 12, wherein the sensor data from the one or more second sensor elements determines which sensor data from the first sensor elements is ignored.

14. A method according to any of the claims 11-13, wherein the sensor data from the one or more second sensor elements determines the orientation of the sensor array, and wherein the sensor data from the first sensor elements is interpreted based on said orientation of the sensor array.

15. A method according to any of the claims 11-14, wherein the one or more second sensor elements comprise temperature sensors, and wherein the sensor data from the one or more second sensor elements is used to determine which sensor data from the first sensor elements relates to a living subject and which sensor data relates to a non-live object.
